Europäisches Patentamt

European Patent Office

Office européen des brevets

(11) Publication number: **0 208 009 B1**

# EUROPEAN PATENT SPECIFICATION

(45) Date of publication of patent specification: **12.06.91**

(21) Application number: **85108562.1**

(22) Date of filing: **10.07.85**

(51) Int. Cl.⁵: **A61K 7/16, A61K 7/20, A61K 7/30**

(54) **Cleansing foam for teeth.**

(43) Date of publication of application:
**14.01.87 Bulletin 87/03**

(45) Publication of the grant of the patent:
**12.06.91 Bulletin 91/24**

(84) Designated Contracting States:
**AT BE CH DE FR GB IT LI NL SE**

(56) References cited:
**DE-A- 2 001 317          FR-A- 1 145 676
FR-A- 2 361 851          GB-A- 1 465 495
US-A- 3 639 568          US-A- 3 822 212
US-A- 3 947 567**

(73) Proprietor: **Richardson GmbH
H.-S.-Richardson-Strasse Postfach 1661
W-6080 Gross-Gerau(DE)**

(72) Inventor: **Agar, Joseph Thomas Henry
2 Bellingham Close Heatherside
Camberley Surrey(GB)**

(74) Representative: **Goddar, Heinz J., Dr. et al
FORRESTER & BOEHMERT Widenmayer-
strasse 4/I
W-8000 München 22(DE)**

## Description

The invention relates to a cleansing foam for teeth in aerosol form, composed of a propellant and a liquid composition comprising an alcohol, a pharmaceutically acceptable surfactant, a pharmaceutically acceptable humectant, a pharmaceutically acceptable bacteriostatic agent, and water.

Cleansers for natural or artificial teeth have conventionally been available in the form of liquids, powders, pastes or effervescent tablets.

A substantially complete removal of plaque or tobacco-film with a known cleansing means is either not obtainable or requires extensive brushing and/or a considerable period of soaking of the dentures. Furthermore, none of the known cleansing means provides an effective activity against candida species and/or a really lasting bacteriostatic activity.

The US-A-4511 486 and GB-A-1 465 495 disclose a cleaning composition for teeth using aerated foams. Although it seems to realise a rapid and effective cleansing, there is no specific improvement in the anti-plaque and /or lasting bacteriostatic acticity.

It is known from a number of publications in various journals (Holbeche, J. D., Rulijancich, M. K., Read, P. C., Austral. Dent. J. 20, 397 (1975); Lobene R. R. et al., Pharmacol. and Therapeutics in Dentistry 4, 33 (1969); Llewelyn, J., Br. Dent. J. 148, 103 (1980), Volpe, A. R. et al.,J. Dent. Res. suppl. 5, 832 (1969)) that cetyl pyridinium chloride may have some anti-plaque activity but at a rather low level. It especially lacks equipotency with chlorhexidine.

Furthermore, all the above applications relate to the use of mouthwash containing cetyl pyridinium chloride. No anti-plaque activity is found when cetyl pyridinium chloride is used in lozenges (Barnes, G. P., Radentz, W. H., Perkins, B. E., J. Prev. Dents. 2, 2 (1975)) or in pastes (Rebstein, F., et al., Acta Paradentol. 10, 51 (1981)).

Thus, the problem of the invention is to provide a cleansing foam for teeth effecting a lasting bacteriostatic and effective anti-plaque activity without extensive brushing and/or a considerable period of soaking of the dentures, in case of using the cleansing foam in its most preferable form, namely for artificial teeth.

According to the invention this problem is solved by a cleansing foam of the above-mentioned kind which is characterized in that the alcohol component is selected from ethanol, isopropanol, and mixtures thereof, wherein its concentration is in the range of 28 to 32 percent by volume; the pharmaceutically acceptable surfactant is a mixture of cetyl stearyl alcohol ethoxylate (20 moles ethylene oxide) and the disodium salt of a dicarboxylic coconut imidazoline derivative, wherein the concentration of the first component of this mixture is in the range of 6.5 to 10.0 percent by weight and the concentration of the second component of this mixture is in the range of 4.5 to 8.0 percent by weight; the pharmaceutically acceptable humectant is glycerol in a concentration of 0.5 to 4.0 percent by weight; and the bacteriostatic agent is cetyl pyridinium chloride in a concentration of 0.55 to 0.70 percent by weight.

Preferred embodiments of the cleansing foam are subject of the respective dependent claims.

The invention further relates to the methods of using the cleansing foam as cleansing means for artificial teeth, i.e. dentures, or as cleansing means for natural teeth, i. e. in a kind of tooth paste.

The cleansing foam according to the invention provides an excellent tobacco film removal of 85 % with a three minute soak or 83 % with brushing of dentures.

The removal of simulated agressive food stain mordanted onto s. mutans plaque shows, with a three minute soak only, equivalent results to a fifteen minute soak in a commercial two phase denture cleanser.

There is found a reduction from $10^8$ to $10^4$ of s. mutans on the whole set of teeth using a one minute brush. Moreover the cleansing foam for teeth according to the invention has a lasting bacteriostatic activity which is unique with dentures.

Furthermore it is effective against candida species. No denture cleansing tablet has this activity.

The cleansing foam for teeth according to the invention does not cause any chemical attack or any damage by brushing with it on fine, highly polished acrylic surfaces or on surfaces of natural teeth.

Surprisingly there has been found a very high plaque inhibiting efficacy when using the cleansing foam according to the invention:

75 % inhibition, by weight, of the growth of a s. mutans plaque on paladon acrylic tiles incubated in a high sucrose medium; 54 % inhibition, by weight, of the growth of the plaque cultured from natural mouth flora on paladon acrylic tiles incubated in a high sucrose medium; significant inhibition of natural plaque on real teeth during an eight hour period using human volunteers; and proven inhibition of s. mutans plaque on prosthesis metal, all dental acrylics and silicon denture liners.

All these advantageous effects can be achieved by 0.55 to 0.70 percent by weight of cetyl pyridinium chloride in the above mentioned liquid composition.

The presence of a surfactant in this liquidcomposition acts to generate the foam and permits penetration of the foam cleanser into the cracks and crevices of the teeth, thereby assisting in the removal of stains, debris and food particles from exposed surfaces. Although several commercially available surfactants can be used, a mixture of a non-ionic type and an amphoteric type detergent is preferred in the present invention whereby the non-ionic type detergent provides the initial rich foaming characteristic to the product while the amphoteric type detergent provides the second foaming generated during the brushing cycle.

In a particularly preferred embodiment of the invention, the surfactant is a mixture of 6.5 - 10.0 percent by weight of the non-ionic type detergent and 4.5 - 8.0 percent by weight of the amphoteric type detergent. The employed detergents are cetyl stearyl alcohol ethoxylate (20 moles ethylene oxide) as a non-ionic type detergent and the disodium salt of an dicarboxylic coconut imidazoline derivative as an amphoteric type detergent.

The alcohol component of the said liquid composition, namely ethanol, has several useful purposes. Firstly, ethanol acts together with cetyl pyridinium chloride to provide an effective anti-microbial action. Secondly, the alcohol component solubilizes the flavouring oils and other adjuvants that may not be water soluble. Thirdly, the alcohol component acts together with a flavouring adjuvant to impart a pleasant fresh taste to the cleansed teeth. The ethanol employed in the present invention ranges from 28 - 32 percent by volume of the said liquid composition.

The presence of a humectant helps to stabilize the foam. A typical water soluble pharmaceutically acceptable humectant, suitable for use in the said liquid composition is glycerol in an amount of 0.5 - 4.0 percent by weight.

Since the cleansing foam for teeth according to the invention is to be stored finally in a metalic can, it is useful to add a corrosion inhibitor to the liquid composition. Sodium nitrate or sodium benzoate, single or in combination, is suitable for this purpose when used in an amount from 0 - 1.0 percent by weight, preferably less than 0.25 percent by weight, in the case of sodium nitrate or in an amount from 0 - 2.0 percent by weight, preferably less than 0.5 percent by weight, in the case of sodium benzoate.

Any colouring and flavouring adjuvants used in cleansing powders or tooth-pastes are also suitable herein. In the preferred embodiment of the present invention a mixture of FD + C Yellow No. 5 in an amount from 0 -0.002 percent by weight and FD + C Green No. 3 in an amount from 0 - 0.0002 percent by weight is selected as colouring adjuvant.

A combination of several essential oils is selected to give the product a pleasant very characteristic aromatic odor when used in an amount from 1.0 - 2.0 percent by weight, preferably 1.4 - 1.9 percent by weight, most preferably 1.80 percent by weight. This special formulation is shown in the following table whereby the amounts are given in percent by weight of the liquid composition relating to the preferred amounts of the ingredients of the flavouring adjuvant:

| | |
|---|---|
| L-carvone | 0.375 |
| anethole | 0.375 |
| cinnamaldehyde | 0.264 |
| menthol | 0.149 |
| peppermint oil | 0.171 |
| 3-hexen-1-ol (leaf alcohol) | 0.0225 |
| ethylene brassylate | 0.00045 |
| additional odorants | 0.3997 |

In order to assure a constantly high quality of the product the liquid composition is filled up to 100 percent by weight with demineralized water and the pH-value is adjusted to a range from 6.5 - 7.5 with nitric acid (20 %). Since the cleansing foam for teeth according to the invention has an excellent cleansing effect, a specific anti-plaque, bacteriostatic activity, a pleasant and fresh colour and flavour and since it is not toxic or agressive, it can be used as cleansing means for artificial as well as for natural teeth.

Further features and advantages of the invention can be gathered from the following description of a performance example.

| | |
|---|---|
| absolute ethanol | 288 ml (228 g) |
| cetyl stearyl alcohol ethoxylate (20 moles ethylene oxide) | 70 g |
| disodium dicarboxylic coconut imidazoline detergent | 50 g |
| glycerol | 10 g |
| cetyl pyridinium chloride | 6 g |
| sodium nitrate | 5 g |
| sodium benzoate | 10 g |
| flavour blend | 18 g |
| FD&C Green No. 3 | 0.0015 g |
| FD&C Yellow No. 5 (tartrazine) | 0.015 g |
| demineralized water | to 1000 ml (add 563 g) |
| nitric acid (20 %) sufficient to adjust pH 7.00 | |

To the alcohol (288 ml) add the cetyl stearyl alcohol ethoxylate (70 g); disodium dicarboxylic coconut imidazoline (50 g) and the flavour blend (18 g); mix until the detergents are dispersed. Add water to approx. half of the final volume and mix until the mixture becomes clear. Add glycerol (10 g), add cetyl pyridinium chloride (6 g) and mix until dissolved.

Into a separate vessel mix the colours FD&C Green No. 3 (0.0015 g), FD&C Yellow No. 5 (0.015 g), sodium nitrate (5 g) and sodium benzoate (10 g), add a small amount of water (100 ml) and mix until dissolved. Add to the main mixture and mix until totally dissolved.

Add water to 90 % of final volume (900 ml), adjust pH of mixture to 7.00 using a 10 % nitric acid solution. Make up to 1 litre with water, mix and store.

An aerosol foam composition is:

| | |
|---|---|
| Product | 92 g |
| Dichlorodifluoromethane | 4 g |
| Dichlorotetrafluoroethane | 4 g |

The features disclosed in the foregoing description, in the following claims and/or in the accompanying drawings may, both separately and in any combination thereof, be material for realising the invention in diverse forms thereof.

## Claims

1. A cleansing foam for teeth in aerosol form, composed of a propellant and a liquid composition comprising an alcohol, a pharmaceutically acceptable surfactant, a pharmaceutically acceptable humectant, a pharmaceutically acceptable bacteriostatic agent, and water,

characterized in that

the alcohol component is selected from ethanol, isopropanol, and mixtures thereof, wherein its concentration is in the range of 28 to 32 percent by volume;

the pharmaceutically acceptable surfactant is a mixture of cetyl stearyl alcohol ethoxylate (20 moles ethylene oxide) and the disodium salt of a dicarboxylic coconut imidazoline derivative, wherein the concentration of the first component of this mixture is in the range of 6.5 to 10.0 percent by weight and the concentration of the second component of this mixture is in the range of 4.5 to 8.0 percent by weight;

the pharmaceutically acceptable humectant is glycerol in a concentration of 0.5 to 4.0 percent by weight; and

the bacteriostatic agent is cetyl pyridinium chloride in a concentration of 0.55 to 0.70 percent by weight.

2. A cleansing foam according to claim 1, characterized in that the alcohol is ethanol.

3. A cleansing foam according to claim 1 or 2, characterized in that a liquid composition is filled up to 100.00 percent by weight with demineralized water.

4. A cleansing foam according to one of the preceding claims, characterized in that the liquid composition further comprises a corrosion inhibitor.

5. A cleansing foam according to claim 4, characterized in that the corrosion inhibitor is sodium nitrate or sodium benzoate or a mixture thereof.

6. A cleansing foam according to claim 5, characterized in that the concentration of a sodium nitrate is between 0.0 and 1.0 percent by weight, preferably less than 0.25 percent by weight, and the concentration of sodium benzoate is between 0.0 and 2.0 percent by weight, preferably less than 0.5 percent by weight.

7. A cleansing foam according to one of the preceding claims, characterized in that the liquid composition further comprises a colouring adjuvant.

8. A cleansing foam according to claim 7, characterized in that the colouring adjuvant is a mixture of FD&C Yellow No. 5 and FD&C Green No. 3.

9. A cleansing foam according to claim 8, characterized in that the concentration of FD&C Yellow No. 5 is in the range of 0.0 to 0.002 percent by weight and the concentration of FD&C Green No. 3 is in the range of 0.0 to 0.0002 percent by weight.

10. A cleansing foam according to one of the preceding claims, characterized in that the liquid composition further comprises a flavouring adjuvant.

11. A cleansing foam according to claim 10, characterized in that the concentration of the flavouring adjuvant is between 1.0 and 2.0 percent by weight, preferably between 1.4 and 1.9 percent by weight, most preferably 1.80 percent by weight.

12. A cleansing foam according to claim 11, characterized in that the flavouring adjuvant has in its preferred formulation (all amounts in percent by weight):

| | |
|---|---|
| L-carvone | 0.375 |
| anethole | 0.375 |
| cinnamaldehyde | 0.264 |
| menthol | 0.149 |
| peppermint oil | 0.171 |
| 3-hexen-1-ol (leaf alcohol) | 0.0225 |
| ethylene brassylate | 0.00045 |
| additional odorants | 0.3997 |

13. A cleansing foam according to one of the preceding claims, characterized in that the pH-value of the liquid composition is adjusted to a range from 6.5 to 7.5.

14. A cleansing foam according to claim 13, characterized in that the pH-value is adjusted with nitric acid.

15. A cleansing foam according to one of the preceding claims, characterized in that the propellant is a mixture of dichlorodifluoromethane and dichlorotetrafluoroethane.

**Revendications**

1. Mousse pour le nettoyage des dents sous forme d'aérosol, composée d'un propulseur et d'une composition liquide comprenant un alcool, un agent tensioactif pharmaceutiquement acceptable, un humectant pharmaceutiquement acceptable, un agent bactériostatique pharmaceutiquement acceptable, et de l'eau, caractérisée par le fait que
   - le composant alcool est choisi parmi l'éthanol, l'isopropanol et leurs mélanges, sa concentration se situant dans la plage de 28 à 32 pour cent en volume ;
   - l'agent tensio-actif pharmaceutiquement acceptable est un mélange d'éthoxylate d'alcool cétylique/stéarylique ( à 20 moles d'oxyde d'éthylène) et du sel disodique d'un dérivé coprah imidazoline dicarboxylique, la concentration du premier composant de ce mélange se situant dans la plage de 6,5 à 10,0 pour cent en poids et la concentration du second composant de ce mélange se situant dans la plage de 4,5 à 8,0 pour cent en poids ;
   - l'humectant pharmaceutiquement acceptable est le glycérol dans une concentration de 0,5 à 4,0 pour cent en poids ; et
   - l'agent bactériostatique est le chlorure de cétyl pyridinium dans une concentration de 0,55 à 0,70 pour cent en poids.

2. Mousse nettoyante selon la revendication 1, caractérisée par le fait que l'alcool est l'éthanol.

3. Mousse nettoyante selon la revendication 1 ou 2, caractérisée par le fait que la composition liquide est complétée jusqu'à 100,00 pour cent en poids par de l'eau déminéralisée.

4. Mousse nettoyante selon l'une des revendications précédentes, caractérisée par le fait que la composition liquide comprend en outre un inhibiteur de corrosion.

5. Mousse nettoyante selon la revendication 4, caractérisée par le fait que l'inhibiteur de corrosion est le nitrate de sodium ou le benzoate de sodium, ou un mélange de ceux-ci.

6. Mousse nettoyante selon la revendication 5, caractérisée par le fait que la concentration du nitrate de sodium se situe entre 0,0 et 1,0 pour cent en poids, étant, de préférence, de moins de 0,25 pour cent en poids, et la concentration du benzoate de sodium se situe entre 0,0 et 2,0 pour cent en poids, étant, de préférence, de moins de 0,5 pour cent en poids.

7. Mousse nettoyante selon l'une des revendications précédentes, caractérisée par le fait que la composition liquide comprend en outre un adjuvant de coloration.

8. Mousse nettoyante selon la revendication 7, caractérisée par le fait que l'adjuvant de coloration est un mélange du Jaune N° 5 FD&C et du Vert N° 3 FD&C.

9. Mousse nettoyante selon la revendication 8, caractérisée par le fait que la concentration du Jaune N° 5 FD&C se situe dans la plage de 0,0 à 0,002 pour cent en poids et la concentration du Vert N° 3 FD&C se situe dans la plage de 0,0 à 0,0002 pour cent en poids.

10. Mousse nettoyante selon l'une des revendications précédentes, caractérisée par le fait que la composition liquide comprend en outre un adjuvant d'aromatisation.

11. Mousse nettoyante selon la revendication 10, caractérisée par le fait que la concentration de l'adjuvant d'aromatisation se situe entre 1,0 et 2,0 pour cent en poids, de préférence, entre 1,4 et 1,9 pour cent en poids, étant, de façon davantage préférée, de 1,80 pour cent en poids.

12. Mousse nettoyante selon la revendication 11, caractérisé par le fait que l'adjuvant d'aromatisation présente, dans sa formulation préférée (toutes les quantités étant pour cent en poids) :

```
L-carvone ................................... 0,375
Anéthole ................................... 0,375
Cinnamaldéhyde ........................... 0,264
Menthol ................................... 0,149
Essence de menthe ........................ 0,171
Hexèn-3 ol-1 (alcool de feuilles) .......... 0,0225
Ethylène brassylate ...................... 0,00045
Substances odorantes additionnelles ........ 0,3997
```

13. Mousse nettoyante selon l'une des revendications précédentes, caractérisée par le fait que le pH de la composition liquide est ajusté à une valeur se situant dans une plage de 6,5 à 7,5.

14. Mousse nettoyante selon la revendication 13, caractérisée par le fait que la valeur du pH est ajustée par de l'acide nitrique.

15. Mousse nettoyante selon l'une des revendications précédentes, caractérisée par le fait que le propulseur est un mélange de dichlorodifluorométhane et de dichlorotétrafluoroéthane.

**Ansprüche**

1. Reinigungsschaum für Zähne in Aerosolform, aus einem Treibmittel und einer flüssigen Zusammensetzung besteht, die einen Alkohol, ein pharmazeutisch akzeptables Tensid, ein pharmazeutische akzeptables Feuchthaltemittel, ein pharmazeutisch akzeptables bakteriostatisches Mittel und Wasser umfaßt;

dadurch gekennzeichnet, daß

die Alkoholkomponente ausgewählt ist aus Ethanol, Isopropanol und Gemischen derselben, wobei ihre Konzentration im Bereich von 28 bis 32 Vol.-% liegt;

das pharmazeutisch akzeptable Tensid ein Gemisch von Cetylstearylalkoholethoxylat (20 Mol Ethylen-

7

oxid) und dem Dinatriumsalz eines Dicarbonsäure-Kokosnußimidazolinderivates ist, wobei die Konzentration der ersten Komponente dieses Gemisches im Bereich von 6,5 bis 10,0 Gew.-% und die Konzentration der zweiten Komponente dieses Gemisches im Bereich von 4,5 bis 8,0 Gew.-% liegt;

das pharmazeutisch akzeptable Feuchthaltemittel Glycerin in einer Konzentration von 0,5 bis 4,0 Gew.-% ist; und

das bakteriostatische Mittel Cetylpyridiniumchlorid in einer Konzentration von 0,55 bis 0,70 Gew.-% ist.

2. Reinigungsschaum nach Anspruch 1, dadurch gekennzeichnet, daß der Alkohol Ethanol ist.

3. Reinigungsschaum nach Anspruch 1 oder 2, dadurch gekennzeichnet, daß die flüssige Zusammensetzung mit entmineralisiertem Wasser auf 100,00 Gew.-% aufgefüllt wird.

4. Reinigungsschaum nach einem der vorangehenden Ansprüche, dadurch gekennzeichnet, daß die flüssige Zusammensetzung außerdem einen Korrosionsinhibitor umfaßt.

5. Reinigungsschaum nach Anspruch 4, dadurch gekennzeichnet, daß der Korrosionsinhibitor Natriumnitrat oder Natriumbenzoat oder ein Gemisch derselben ist.

6. Reinigungsschaum nach Anspruch 5, dadurch gekennzeichnet, daß die Konzentration von Natriumnitrat zwischen 0,0 und 1,0 Gew.-%, vorzugsweise weniger als 0,25 Gew.-%, und die Konzentration von Natriumbenzoat zwischen 0,0 und 2,0 Gew.-%, vorzugsweise weniger als 0,5 Gew.-%, liegt.

7. Reinigungsschaum nach einem der vorangehenden Ansprüche, dadurch gekennzeichnet, daß die flüssige Zusammensetzung weiter einen Farbzusatz umfaßt.

8. Reinigungsschaum nach Anspruch 7, dadurch gekennzeichnet, daß der Farbzusatz ein Gemisch von FD&C Gelb Nr. 5 und FD&C Grün Nr. 3 ist.

9. Reinigungsschaum nach Anspruch 8, dadurch gekennzeichnet, daß die Konzentration von FD&C Gelb Nr. 5 im Bereich von 0,0 bis 0,002 Gew.-% und die Konzentration von FD&C Grün Nr. 3 im Bereich von 0,0 bis 0,0002 Gew.-% liegt.

10. Reinigungsschaum nach einem der vorangehenden Ansprüche, dadurch gekennzeichnet, daß die flüssige Zusammensetzung außerdem einen Geschmackszusatz umfaßt.

11. Reinigungsschaum nach Anspruch 10, dadurch gekennzeichnet, daß die Konzentration des Geschmackszusatz zwischen 1,0 und 2,0 Gew.-%, vorzugsweise zwischen 1,4 und 1,9 Gew.-%, am meisten bevorzugt 1,80 Gew.-% ist.

12. Reinigungsschaum nach Anspruch 11, dadurch gekennzeichnet, daß der Geschmackszusatz in einer bevorzugten Formulierung folgendes umfaßt (alle Mengen in Gew.-%):

| | |
|---|---|
| L-Carvon | 0,375 |
| Anethol | 0,375 |
| Zimtaldehyd | 0,264 |
| Menthol | 0,149 |
| Pfefferminzöl | 0,171 |
| 3-Hexen-1-öl (Blattalkohol) | 0,0225 |
| Ethylenbrassylat | 0,00045 |
| Zusätzliche Duftstoffe | 0,3997 |

13. Reinigungsschaum nach einem der vorangehenden Ansprüche, dadurch gekennzeichnet, daß der pH-Wert der flüssigen Zusammensetzung auf einen Bereich von 6,5 bis 7,5 eingestellt ist.

14. Reinigungsschaum nach Anspruch 13, dadurch gekennzeichnet, daß der pH-Wert mit Salpetersäure eingestellt ist.

15. Reinigungsschaum nach einem der vorangehenden Ansprüche, dadurch gekennzeichnet, daß das Treibmittel ein Gemisch von Dichlordifluormethan und Dichlortetrafluorethan ist.